# EUROPÄISCHE PATENTSCHRIFT

(11) **EP 0 607 802 B1**
(45) Veröffentlichungstag und Bekanntmachung des Hinweises auf die Patenterteilung: **22.04.1998**
(21) Anmeldenummer: 94100105.9
(22) Anmeldetag: 05.01.1994
(51) Int. Cl.: C07C 253/30, C07C 255/30

(54) **Verfahren zur Herstellung eines Gemisches von aminomethylenierten Glutaconsäuredinitrilen**
Process for the preparation of a mixture of aminomethylenated glutacononitriles
Procédé pour la préparation d'une mélange de dinitriles de l'acide glutaconique aminométhylènés

(30) Priorität: 19.01.1993 DE 4301238
(43) Veröffentlichungstag der Anmeldung: 27.07.1994
(73) Patentinhaber: BAYER AG, 51368 Leverkusen (DE)
(72) Erfinder: Kraus, Helmut, Dr., D-51519 Odenthal (DE)

(56) Entgegenhaltungen:
- US-A- 3 138 631
- JOURNAL OF ORGANIC CHEMISTRY. Bd. 29 , 1964 , WASHINGTON DC US Seiten 1800 - 1808 F. SCOTTI ET AL. 'The synthesis and Reactions of beta-Chloroacrylonitrile'
- JOURNAL OF THE CHEMICAL SOCIETY, (C). 1969 , LETCHWORTH GB Seiten 1086 - 1088 T. SASAKI ET AL. 'The Chemistry of Cyanoacetylenes. Part II. Cyanoenamines, their Preparation and Reactions'
- Dissertation H. Saur, Univ. Stuttgart 1971

## Beschreibung

Die Erfindung betrifft ein Verfahren zur Herstellung von 5-Amino-4-cyano-pentadien-2,4-nitrilen (aminomethylenierten Glutaconsäuredinitrilen) durch Dimerisierung der zugrundeliegenden 3-Amino-acrylsäurenitrile, wobei die Titelverbindungen in Form von Gemischen anfallen, wenn man von verschieden substituierten 3-Amino-acrylsäurenitrilen ausgeht.

Die anspruchsgemäß herstellbaren Verbindungen sind wichtige Zwischenprodukte zur Herstellung von Heterocyclen. Besonders elegant ist der Ringschluß zu 2,5-disubstituierten Pyridinen, die zur Herstellung von Herbiziden (EP 483) oder Insektiziden (EP 235 725) benötigt werden. 5-Amino-4-cyano-pentadien-2,4-nitrile sollten sich prinzipiell analog EP 268 964 durch Umsetzung von Glutaconsäuredinitril mit o-Amiden herstellen lassen. Allerdings sind Glutaconsäurederivate nur aufwendig herstellbar; technische Verfahren sind zur Zeit nicht bekannt.

Aus J. Chem. Soc. (C) 1969, 1086-1088 war bekannt, daß die Reaktion von Aminonitrilen mit Acylchloriden über ein Zwischenprodukt zu Diendinitrilen führt. Für die Morpholin-substituierte Verbindung werden 75 % Ausbeute (in Trichloressigsäure) bzw. von 33 % (in Monochloressigsäure) angegeben. Reaktionsdaten für Dimethylamino-subsitutierte Verbindungen werden nicht offenbart.

In EP 162 464 wird ein C₆-Pyridinvorläufer aus einem C₃-Enamin und α-Chloracrylnitril synthetisiert. Bei Verwendung identischer C₃-Amine ist die C₆-Bildung, d.h. die gewünschte Dimerisierung, jedoch nur ein Zwischenschritt in Richtung auf weitergehende und unerwünschte Bildung von Oligomeren bzw. Polymeren; der Zwischenschritt ist nicht kontrollierbar und daher technisch nicht zu nutzen.

In J. Org. Chem. 29 (1964), 1800 wird eine Dimerisierung von Dimethylaminoacrylnitril in Gegenwart von Eisessig beschrieben. Die Ausbeute von 40 % d.Th. läßt allerdings zu wünschen übrig.

Die Dimerisierung von 3-Dimethylamino-acrylnitril in Gegenwart von Schwefelsäure gelingt in immer noch unzureichenden 51 % der theoretischen Ausbeute, wenn der Reaktionsansatz nach 4 Minuten unterbrochen und aufgearbeitet wird (Dissertation M. Saur, Univ. Stuttgart 1971). Eine solche Reaktionsführung ist technisch jedoch nur mit sehr viel Aufwand und in unwirtschaftlich kleinen Ansätzen zu realisieren.

Von daher war es überraschend, daß ein Verfahren gefunden werden konnte, daß es erlaubt, 5-Amino-4-cyanopentadien-2,4-nitrile durch selektive Dimerisierung der zugrundeliegenden und entsprechend substituierten Acrylnitrile herzustellen. Kennzeichnend für das erfindungsgemäße Verfahren ist die hohe Reinheit der Reaktionsprodukte, die in über 90 % der theoretischen Ausbeute erhalten werden. Andererseits werden Folgereaktionen vermieden, so daß eine normale Isolierung und Aufarbeitung, wie beispielsweise durch Filtration über eine Nutsche, möglich ist.

Die Erfindung betrifft demnach ein Verfahren zur Herstellung eines Gemisches von aminomethylenierten Glutaconsäuredinitrilen der Formeln 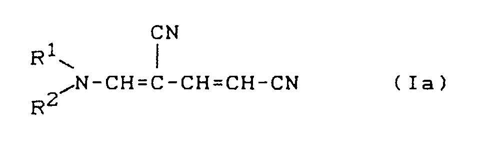 bzw. 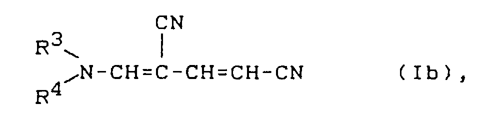 worin
- R¹, R², R³ und R⁴: unabhängig voneinander Wasserstoff, geradkettiges oder verzweigtes C₁-C₈-Alkyl, C₃-C₈-Alkenyl, C₂-C₈-Alkoxyalkyl, C₄-C₈-Alkoxyalkenyl, C₃-C₈-Cycloalkyl, C₆-C₁₂-Aryl, C₇-C₁₀-Aralkyl oder einen 5- bis 8-gliedrigen gesättigten oder ungesättigten heterocyclischen Ring, dessen Heteroatome 1 oder 2 aus der Gruppe von N, O und S sind, darstellen,
das dadurch gekennzeichnet ist, daß man 3-Amino-acrylnitrile der Formeln 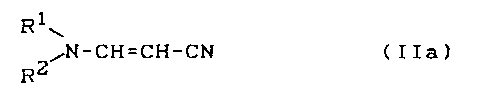 bzw. 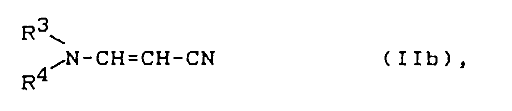 in denen R¹ bis R⁴ den genannten Bedeutungsumfang haben,

in Gegenwart von 0,5 bis 50 Äquivalenten einer sauren Verbindung pro Mol der Gesamtmenge der 3-Amino-acrylnitrile miteinander umsetzt, wobei in einem organischen Verdünnungsmittel gearbeitet wird.

Bei der Dimerisierung nach dem erfindungsgemäßen Verfahren werden in grundsätzlich gleichberechtigter Reaktion sowohl das Amin HN(R¹, R²) als auch HN(R³, R⁴) als Spaltprodukte eliminiert. So kann das Gemisch der erfindungsgemäß erhaltenen Reaktionsprodukte in der Nähe von je 50 Mol-% für die beiden Substanzen (Ia) und (Ib) liegen. Bei starker Unterschiedlichkeit der Substituenten R¹ bis R⁴ kann jedoch eines der beiden herausgespaltenen Amine überwiegen. So wird beobachtet, daß beim Vorhandensein aromatischer Gruppen im Rahmen des Bedeutungsumfanges von R¹ bis R⁴ sich diese aromatischen Gruppen auch im Reaktionsprodukt wiederfinden, während die nicht-aromatischen Gruppen in Form des Amins herausgespalten werden. Die Reaktionsprodukte der Formeln (Ia) und (Ib) können durch fraktionierte Destillation, Kristallisation, Chromatographie und andere Methoden getrennt werden. Für den Fall, daß das als Ausgangsmaterial eingesetzte 3-Amino-acrylnitril mit den Substituenten R³ und R⁴ schwierig herstellbar ist, kann das erfindungsgemäße Verfahren auch in Anwesenheit eines Amins der Formel 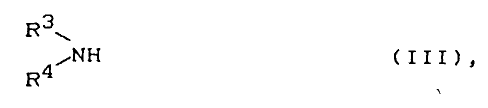 in der
R³ und R⁴ den obigen Bedeutungsumfang haben,
hergestellt werden. In einem solchen Fall wird regelmäßig ein Aminaustausch zwischen dem Ausgangsmaterial (IIa) und dem Amin (III) beobachtet, so daß in ausreichender Menge Reaktionsprodukt der Formel (Ib) erhalten werden kann.

In vielen Fällen wird man jedoch bestrebt sein, Gemische der Reaktionsprodukte (Ia) und (Ib), die eine zusätzliche Auftrennung erfordern, zu vermeiden. In einem solchen Fall werden Startmaterialien eingesetzt, in denen R³ identisch mit R¹ und R⁴ identisch mit R² sind. In anderen Worten: als Startmaterial wird lediglich ein solches der Formel (IIa) eingesetzt, und als Reaktionsprodukt wird lediglich ein solches der Formel (Ia) erhalten.

Geradkettiges oder verzweigtes C₁-C₈-Alkyl ist beispielsweise Methyl, Ethyl, Propyl, Isopropyl, Butyl, Isobutyl, tert.-Butyl, die isomeren Pentyle, Hexyle, Octyle, bevorzugt die genannten C₁-C₄-Alkylreste.

Geradkettiges oder verzweigtes C₃-C₈-Alkenyl bedeutet beispielsweise die isomeren Butenyle, Pentenyle, Hexenyle oder Octenyle, bevorzugt die genannten C₃-C₄-Alkenylreste.

Geradkettiges oder verzweigtes C₂-C₈-Alkoxyalkyl ist beispielsweise Methoxymethyl, Ethoxymethyl sowie weitere Reste aus der Gruppe C₃-C₉-Alkyl, in welchen eine CH₂-Gruppe durch ein O-Atom ersetzt ist.

Geradkettiges oder verzweigtes C₄-C₈-Alkoxyalkenyl ist beispielsweise Methoxyallyl, 2-Methoxy-propenyl und andere aus der Gruppe von C₄-C₉-Alkenyl, worin eine CH₂-Gruppe durch ein O-Atom ersetzt ist.

C₃-C₈-Cycloalkyl ist beispielsweise Cyclopropyl, Methylcyclopropyl, Dimethyl-cyclopropyl, Cyclobutyl, Methylcyclobutyl, Cyclopentyl, Methyl-cyclopentyl, Cyclohexyl, Methyl-cyclohexyl, Dimethyl-cyclohexyl, Cycloheptyl, Cyclooctyl, bevorzugt Cyclopropyl, Cyclopentyl und Cyclohexyl, sowie deren Methyl- oder Dimethyl-Derivate.

C₆-C₁₂-Aryl ist beispielsweise Phenyl, Naphthyl oder Biphenylyl, bevorzugt Phenyl.

C₇-C₁₀-Aralkyl ist beispielsweise Benzyl, 1-Phenylethyl, 2-Phenylethyl und weitere dem Fachmann bekannte Reste dieser Art, bevorzugt Benzyl.

Als 5- bis 8-gliedriger gesättigter oder ungesättigter heterocyclischer Ring, dessen Heteroatome 1 oder 2 aus der Gruppe von N, O und S sind, seien genannt: Pyrrol, Furan, Thiophen, Pyrrolidin, Pyrazol, Imidazol, Thiazol, Oxazol, Pyridin, Pyrimidin, Piperazin, das am N-Atom durch C₁-C₄-Alkyl oder durch Hydroxy-C₁-C₄-alkyl substituiert sein kann, Morpholin, Pyran, Azepin, Azocin, Isoxazol, Isothiazol, Pyridazin und Pyrazin. Es ist dem Fachmann bekannt, daß ungesättigte heterocyclische Ringe einen mehr oder weniger stark ausgeprägten aromatischen Charakter haben können. In bevorzugter Weise seien als solche gesättigte oder ungesättigte heterocyclische Ringe Morpholin, Pyrrolidin und Piperidin, das durch C₁-C₄-Alkyl oder durch Hydroxy-C₁-C₄-alkyl substituiert sein kann, genannt.

Die als Startmaterialien erforderlichen substituierten β-Amino-acrylnitrile sind durch Umsetzung von Salzen des Formylpropionsäurenitrils mit sekundären Aminen (EP 18 473) oder durch Kondensation von o-Amiden mit Acetonitril leicht zugänglich.

Die erfindungsgemäße Umsetzung erfolgt bei einer Temperatur von 0 bis 100°C, bevorzugt 10 bis 60°C.

Die erfindungsgemäße Umsetzung erfolgt ferner in Gegenwart von 0,5 bis 50 Äquivalenten, bevorzugt 1 bis 10 Äquivalenten pro Mol der Gesamtmenge an Ausgangsmaterialien der Formel (IIa) und (IIb).

Das erfindungsgemäße Verfahren kann in der bevorzugten Form, daß R¹ und R³ sowie R² und R⁴ identisch sind, formelmäßig wie folgt dargestellt werden:

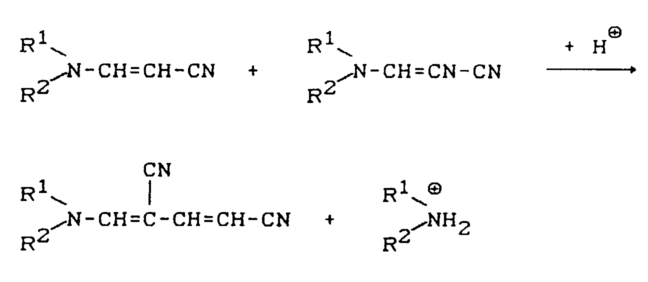

Als erfindungsgemäß einsetzbare saure Verbindungen kommen organische und anorganische Säuren sowie deren Addukte mit polaren organischen Verbindungen in Frage. In bevorzugter Weise werden organische Säuren oder Addukte von anorganischen Säuren mit polaren organischen Verbindungen eingesetzt.

Beispiele für Addukte von anorganischen Säuren mit polaren organischen Verbindungen sind: Dimethylformamid (DMF)/HCl, DMF/H₂SO₄, Acetonitril/HCl, Dimethylacetamid/HCl, Aceton/HCl. Unter diesen Komplexen sind einige nur in Lösung beständig; in bevorzugter Weise werden jedoch die Komplexe eingesetzt, die als Substanzen isolierbar sind.

Als organische Säuren kommen vor allem aliphatische C₁-C₆-Carbonsäuren und deren Halogen- oder C₁-C₄-Alkoxy-Derivate in Frage. Solche Säuren sind beispielsweise: Ameisensäure, Essigsäure, Propionsäure, Buttersäure, Dichloressigsäure, Methoxyessigsäure und analog aufgebaute, dem Fachmann wohl bekannte Substanzen. Diese Säuren werden bevorzugt als wasserfreie Substanzen eingesetzt. Um die Abwesenheit von Wasser sicherzustellen, kann eine geringe Menge des zugehörigen Anhydrids zugesetzt werden.

Geeignete organische Verdünnungsmittel umfassen solche aus der Gruppe der Kohlenwasserstoffe, der halogenierten Kohlenwasserstoffe, der Ketone, Amide, Nitrile, Ether oder Ester. Beispiele hierfür sind: Toluol, Xylol, Chlorbenzol, Ligroin, Aceton, Methylethylketon, Acetonitril, Chloroform, Methylenchlorid, Dichlorethan, Essigsäuremethylester, Essigsäurebutylester, Methyl-tert.-butylether, tert.-Amylmethylether, Dimethylformamid (DMF), N-Methylpyrrolidon (NMP), N-Methylcaprolactam (NMC), Tetramethylharnstoff und andere, dem Fachmann wohl bekannte Substanzen.

Für den Fall, daß als saure Verbindung in bevorzugter Weise eine aliphatische C₁-C₆-Carbonsäure oder deren Halogen- oder Alkoxy-Derivat eingesetzt wird, können solche Säuren auch als Reaktionsmedium dienen, so daß auf ein zusätzliches Verdünnungsmittel verzichtet werden kann. In diesem Fall werden die Säuren in einer Menge eingesetzt, die im oberen Teil des obengenannten Bereiches liegt.

Wie in der Beschreibungseinleitung ausgeführt wurde, eignen sich die erfindungsgemäß hergestellten Glutaconsäuredinitrile zum Ringschluß zu 2,5-disubstituierten Pyridinen. Das Reaktionsmedium für das erfindungsgemäße Verfahren (saure Verbindung und organisches Verdünnungsmittel oder organische Säure ohne ein zusätzliches Verdünnungsmittel) werden daher vorteilhaft so gewählt, daß diese Cyclisierung sofort darin vorgenommen werden kann. Beispiele hierfür sind: DMF/HCl und DMF/H₂SO₄. In einer weiteren vorteilhaften Variante wird das Reaktionsmedium so gewählt, daß das Glutaconsäuredinitril darin in Form einer noch gut rührfähigen Suspension entsteht und nach Ende der Reaktion gut isoliert werden kann. Zu einer solchen Isolierung saugt man das ausgefallene Reaktionsprodukt ab, engt die dabei entstehende Mutterlauge weitgehend ein und wäscht mit Wasser. Dabei fällt weiteres Reaktionsprodukt in ebenfalls hoher Reinheit aus. Ebensogut kann man jedoch den rohen Reaktionsansatz vollständig einengen und mit Wasser versetzen, wobei wiederum das Reaktionsprodukt in fester Form anfällt.

Für die Reaktionsführung und die Ausbeute ist es nicht entscheidend, ob man von reinem trans-Amino-alkylnitril ausgeht oder von Gemischen, die herstellungsbedingt einen Anteil des cis-Isomeren enthalten.

Zur Herstellung von aminomethylenierten Glutaconsäuredinitrilen mit vorbestimmten Aminsubstituenten kann man einerseits das entsprechend substituierte Amino-acrylnitril verwenden. In der oben beschriebenen Weise ist technisch hergestellte 3-Dimethylamino-acrylnitril mit dem gewünschten Amin der Formel (III) umzusetzen. Der Einsatz von Anilin in dieser Variante führt beispielsweise zu 5-Phenyl-amino-4-cyan-pentadien-2,4-nitril, einem Vorläufer für das entsprechende N-Phenyl-pyridon.

### Beispiel 1

192 g 99,5 %iges 3-Dimethylaminoacrylnitril (enthält 0,3 % DMF; 98,2 % trans-, 1,8 % cis-Verbindung) pumpte man in 2 l Eisessig. Der Ansatz mußte während der ersten 15 Minuten durch leichte Kühlung auf Raumtemperatur gehalten werden. Nach 17 h Rühren wurde die Lösung am Rotationsverdampfer bei 14 mbar und bis 50°C Sumpftemperatur eingeengt. Den Rückstand verrührte man mit 1,3 l Wasser, saugte ab und wusch mit 200 ml Wasser nach. Nach Trocknen bei 50°C (300 mbar) wurden 129,3 g schwach orange-farbiges Pulver erhalten, entsprechend 88,4 % der theoretischen Ausbeute. Die Mutterlauge wurde mit Methylenchlorid extrahiert und eingeengt; es wurden weitere 4,2 % an Produkt erhalten.

N,N-Dimethylaminomethylen-glutaconsäuredinitril:
Smp.: 121-122°C
¹H-NMR (d-DMSO): 3,2 ppm, d (J = 28 Hz), 6 H; 4,95 ppm, d (J = 15 Hz), 1 H; 7,18 ppm, 1 (J = 15 Hz), 1 H; 7,47 ppm, s, 1 H

### Beispiel 2

3 Mol 3-Dimethylaminoacrylnitril (92 % trans, 8 % cis) wurden in 1620 ml Eisessig dimerisiert. Die Suspension wurde eingeengt, mit Wasser versetzt und abgesaugt. Man erhielt 199,2 g 98,9 %ige Ware, entsprechend 89,3 % der theoretischen Ausbeute. Die Mutterlauge enthielt weitere 3,6 % an Produkt.

### Beispiel 3

9,6 g 3 3-Dimethylaminoacrylnitril wurden zu 30 ml Eisessig getropft, 14 h gerührt und die Suspension abgesaugt. Man erhielt 5,4 g gelben Feststoff, entsprechend 73,5 % der theoretischen Ausbeute. Nach Einengen der Mutterlauge und Wasserzugabe konnten weitere 1,3 g isoliert werden, entsprechend einer Gesamtausbeute von 91,2 %.

### Beispiel 4

Analog Beispiel 3 tropfte man 0,1 Mol 3-Dimethylaminoacrylnitril zu einer Mischung aus 54 ml Eisessig und 5 ml Acetanhydrid. Man erhielt 2,3 g orangefarbene nadelförmige Kristalle und nach Einengen und Verwässern 4,5 g orangefarbenes Pulver. Die eingeengte Mutterlauge (4,5 g) enthielt 3,4 % an Dimer; d.h. 92,5 % isolierte Ausbeute bzw. 94,7 % Gesamtausbeute. Als Nebenkomponente wurde in 1,1 % der theoretischen Ausbeute 1,3,5-Tricyanobenzol identifiziert.

### Beispiel 5

9,6 g 3-Dimethylaminoacrylnitril und 9,3 g Anilin wurden zu 50 ml Eisessig getropft. Dabei stieg die Temperatur auf 40°C. Man ließ über Nacht stehen und saugte die orangefarbene Suspension ab. Man erhielt 6,8 g 5-Phenylaminomethylen-glutaconsäuredinitril, entsprechend 69,7 % der theoretischen Ausbeute.
¹H-NMR (d-DMSO): 5,23 ppm (d, 16 Hz, 1H); 7,08 bis 7,43 ppm (m, 6H); 8,25 ppm (d, 16 Hz, 1H); 10,55 ppm (d, 14 Hz, 1 H)

### Beispiel 6

Zu einer Lösung von 11 g DMF-HCl-Addukt in 50 ml DMF tropfte man 9,6 g 3-Dimethylaminoacrylnitril. Die Temperatur wurde anfangs durch schwaches Kühlen auf 30°C gehalten. Nach 5 h Rühren bei Raumtemperatur engte man die hellgelbe Suspension ein, versetzte mit 20 ml Wasser und saugte ab. Man erhielt 6,55 g 96,2 %iges Produkt, entsprechend 91,0 % der theoretischen Ausbeute.

### Beispiel 7

Eine Lösung von 28,8 g 3-Dimethylaminoacrylnitril in 180 ml Eisessig wurde 2 Stunden auf 50°C erhitzt und anschließend bei dieser Temperatur im Vakuum eingeengt. Nach Verwässern und Aufarbeitung wurden das Produkt in einer Ausbeute von 87 % d.Th. erhalten.

## Patentansprüche

1. Verfahren zur Herstellung eines Gemisches von aminomethylenierten Glutaconsäuredinitrilen der Formeln 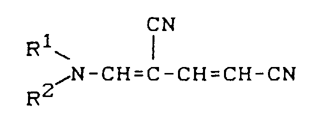 bzw. 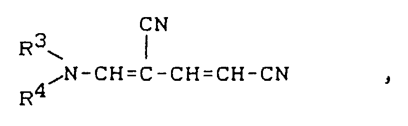 worin
R¹, R², R³ und R⁴ unabhängig voneinander Wasserstoff, geradkettiges oder verzweigtes C₁-C₈-Alkyl, C₃-C₈-Alkenyl, C₂-C₈-Alkoxyalkyl, C₄-C₈-Alkoxyalkenyl, C₃-C₈-Cycloalkyl, C₆-C₁₂-Aryl, C₇-C₁₀-Aralkyl oder einen 5- bis 8-gliedrigen gesättigten oder ungesättigten heterocyclischen Ring, dessen Heteroatome 1 oder 2 aus der Gruppe von N, O und S sind, darstellen,
dadurch gekennzeichnet, daß man 3-Amino-acrylnitrile der Formeln 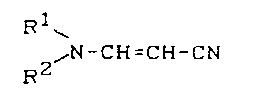 bzw. 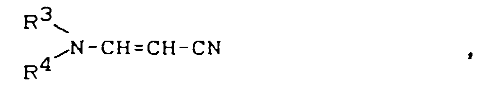 in denen R¹ bis R⁴ den genannten Bedeutungsumfang besitzen,
bei 0 bis 100°C in Gegenwart von mindestens 0,5 Äquivalenten einer sauren Verbindung pro Mol der Gesamtmenge der 3-Amino-acrylnitrile miteinander umsetzt, wobei in einem organischen Verdünnungsmittel gearbeitet wird.

2. Verfahren nach Anspruch 1, dadurch gekennzeichnet, daß anstelle des 3-Amino-acrylnitrils der Formel 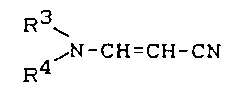 ein Amin der Formel 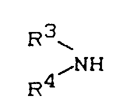 eingesetzt wird, wobei R³ und R⁴ den in Anspruch 1 gegebenen Bedeutungsumfang haben.

3. Verfahren nach Anspruch 1, dadurch gekennzeichnet, daß R³ identisch mit R¹ und R⁴ identisch mit R² sind.

4. Verfahren nach Anspruch 1, dadurch gekennzeichnet, daß als saure Verbindungen organische Säuren eingesetzt werden.

5. Verfahren nach Anspruch 4, dadurch gekennzeichnet, daß aliphatische C₁-C₆-Carbonsäuren und deren Halogen- oder C₁-C₄-Alkoxy-Derivate als Säuren eingesetzt werden.

6. Verfahren nach Anspruch 1, dadurch gekennzeichnet, daß Addukte polarer organischer Verbindungen und anorganischer Säuren als saure Verbindungen eingesetzt werden.

7. Verfahren nach Anspruch 1, dadurch gekennzeichnet, daß mindestens 1 Äquivalent der sauren Verbindung eingesetzt wird.

8. Verfahren nach Anspruch 1, dadurch gekennzeichnet, daß bei 10 bis 60°C gearbeitet wird.

## Claims

1. Process for the preparation of a mixture of aminomethylenated glutaconic acid dinitriles of the formulae 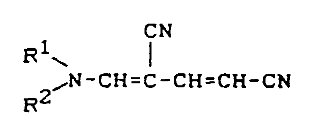 and 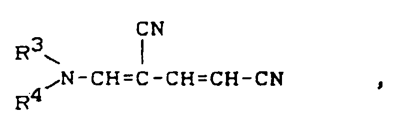 in which
R¹, R², R³ and R⁴ independently of one another represent hydrogen, straight-chain or branched C₁-C₈-alkyl, C₃-C₈-alkenyl, C₂-C₈-alkoxyalkyl, C₄-C₈-alkoxyalkenyl, C₃-C₈-cycloalkyl, C₆-C₁₂-aryl, C₇-C₁₀-aralkyl or a 5- to 8-membered, saturated or unsaturated heterocyclic ring containing 1 or 2 heteroatoms from the group of N, O and S,
characterized in that 3-amino-acrylonitriles of the formulae 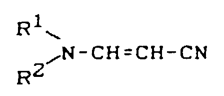 and 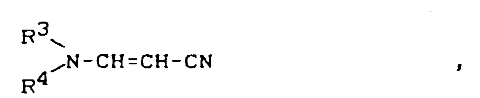 in which R¹ to R⁴ have the scope of meaning mentioned,
are reacted with one another at from 0 to 100°C at least in the presence of 0.5 equivalent of an acidic compound per mole of the total quantity of 3-aminoacrylonitriles, the reaction being carried out in an organic diluent.

2. Process according to Claim 1, characterized in that, instead of the 3-amino-acrylonitrile of the formula 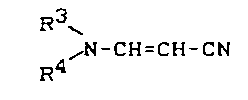 an amine of the formula 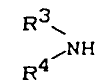 is employed in which R³ and R⁴ have the scope of meaning given in Claim 1.

3. Process according to Claim 1, characterized in that R³ is identical with R¹ and R⁴ is identical with R².

4. Process according to Claim 1, characterized in that the acidic compounds employed are organic acids.

5. Process according to Claim 4, characterized in that the acids employed are aliphatic C₁-C₆-carboxylic acids and their halogeno or C₁-C₄-alkoxy derivatives.

6. Process according to Claim 1, characterized in that the acidic compounds employed are adducts of polar organic compounds and inorganic acids.

7. Process according to Claim 1, characterized in that at least 1 equivalent of the acidic compound is employed.

8. Process according to Claim 1, characterized in that the reaction is carried out at from 10 to 60°C.

## Revendications

1. Procédé pour la préparation d'un mélange de dinitriles glutaconiques aminométhylénés de formules respectives : 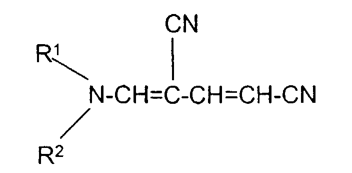 et 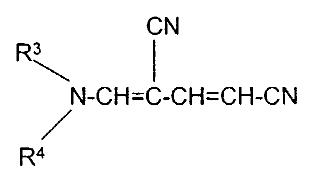 dans lesquelles :
R¹, R², R³ et R⁴ représentent chacun, indépendamment les uns des autres, l'hydrogène, un groupe alkyle en C₁-C₈ à chaîne droite ou ramifiée, alcényle en C₃-C₈, alcoxyalkyle en C₂-C₈, alcoxyalcényle en C₄-C₈, cycloalkyle en C₃-C₈, aryle en C₆-C₁₂, aralkyle en C₇-C₁₀, ou un noyau hétérocyclique saturé ou insaturé de 5 à 8 chaînons contenant 1 ou 2 hétéroatomes choisis parmi N, O et S,
caractérisé en ce que l'on fait réagir entre eux des 3-aminoacrylonitriles de formules respectives : 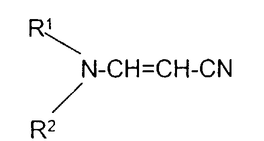 et 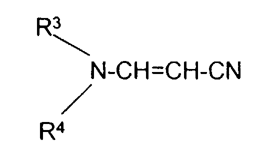 dans lesquelles R¹ à R⁴ ont les significations indiquées ci-dessus,
à des températures de 0 à 100°C en présence d'au moins 0,5 équivalent d'un composé acide par mole des 3-aminoacrylonitriles totaux, la réaction étant réalisée dans un diluant organique.

2. Procédé selon la revendication 1, caractérisé en ce que, à la place du 3-aminoacrylonitrile de formule : 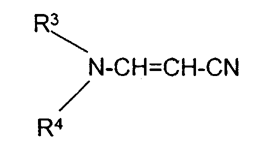 on met en oeuvre une amine de formule : 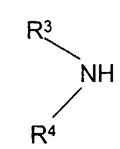 dans laquelle R³ et R⁴ ont les significations indiquées dans la revendication 1.

3. Procédé selon la revendication 1, caractérisé en ce que R³ est identique à R¹ et R⁴ est identique à R².

4. Procédé selon la revendication 1, caractérisé en ce que l'on utilise en tant que composé acide un acide organique.

5. Procédé selon la revendication 4, caractérisé en ce que l'on utilise en tant qu'acide un acide carboxylique aliphatique en C₁ à C₆ ou l'un de ses dérivés halogénés ou portant des substituants alcoxy en C₁ à C₄.

6. Procédé selon la revendication 1, caractérisé en ce que l'on utilise en tant que composé acide un adduct d'un composé organique polaire et d'un acide minéral.

7. Procédé selon la revendication 1, caractérisé en ce que l'on utilise au moins un équivalent du composé acide.

8. Procédé selon la revendication 1, caractérisé en ce que l'on opère à des températures de 10 à 60°C.
